# EUROPEAN PATENT SPECIFICATION

(11) **EP 4 230 113 B1**
(45) Date of publication and mention of the grant of the patent: **10.12.2025**
(21) Application number: 22156963.5
(22) Date of filing: 16.02.2022
(51) Int. Cl.: A61B 5/00, A61B 5/12, A61B 5/369, A61B 5/398, G02B 27/01, G06F 3/01

(54) **DEVICE FOR DETERMINING A HEARING CAPACITY OF A PERSON**
VORRICHTUNG ZUR BESTIMMUNG DER HÖRFÄHIGKEIT EINER PERSON
DISPOSITIF DE DÉTERMINATION DE LA CAPACITÉ AUDITIVE D'UNE PERSONNE

(43) Date of publication of application: 23.08.2023
(73) Proprietor: MiMi Holding B.V., 2289 CC Rijswijk (NL)
(72) Inventor: MAJID, Parzijn, Rijswijk (NL)
(74) Representative: Algemeen Octrooi- en Merkenbureau B.V.

(56) References cited:
- EP-A1- 3 456 259
- WO-A1-2018/218356
- WO-A1-2022/013566

## Description

The present invention relates to a device for determining a hearing capacity of a person, as well as a method, not part of the invention, for determining a hearing capacity of a person using such a device.

### PRIOR ART

Hearing loss, especially untreated hearing loss, constitutes a great risk to humanity. It is estimated that in 2050 1 out of 4 persons will suffer from insufficient hearing capacity. It is further estimated that 2 out 3 persons are not treated satisfactorily for hearing loss. This may lead to fatigue, depression, loneliness, unemployment, in short: much lower quality of life. For the persons that do get treated, for instance by being provided with a hearing aid, audio quality and speech intelligibility are sub-par. This is basically due to the fact that the standard diagnostic methods used for determining the hearing capacity of the person are essentially over 90 years old. The standard diagnostic method for determining hearing capacity can generally be seen as inaccurate, having "low resolution" and lacking in view of dealing with spatial sound localization issues.

WO 2019/010540 A1 discloses a method for assessing the hearing of a patient using functional near-infrared spectroscopy (fNIRS), the method comprising: receiving at least one response signal from an optode placed on a scalp of the patient, the response signal comprising fNIRS data generated by the optode and relating to an aural stimulation received by the patient; comparing at least one parameter of the at least one response signal to a predetermined parameter value; and determining an auditory response of a patient based on the at least one parameter of the at least one response signal. The at least one response signal comprises signals relating to brain activity of the patient. However, merely determining an auditory response of the patient based on his or her brain activity may still lead to an incomplete picture of the patient's hearing capacity, which is particularly troublesome when a hearing aid is to be prescribed.

US 2020/0315499 A1 discloses means for determining acoustic immittance and other characteristics of ears by measuring eardrum displacements resulting from application of pressure to the eardrum. For example, optical coherence tomography may be applied to monitor eardrum displacements responsive to a sound. The pressure corresponding to the sound is measured by a suitable instrument such as a microphone. The measured displacements and pressures may be processed to obtain a measure of immitance. US 2020/0315499 A1 thus also primarily focusses on what happens in the ear canal/middle ear and leaves a lot to be desired when determining various aspects of the person's hearing capacity, in particular when a tailor-made hearing aid is to be prescribed.

EP 3 456 259 A1 discloses an apparatus for adjusting a hearing aid device solely while the hearing aid device is worn by a person.

Thus, the currently available diagnostic processes and/or available diagnostic instruments do not provide enough information to determine multiple/various aspects of the person's hearing capacity, especially when a tailor-made hearing aid is to be prescribed and fitted.

### OBJECT OF THE INVENTION

An object of the present invention is thus to improve the current diagnostic processes and/or available diagnostic instruments for determining multiple/various aspects of the person's hearing capacity, especially when a tailor-made hearing aid is to be prescribed and fitted.

### SUMMARY OF THE INVENTION

According to the present invention a device for determining a hearing capacity of a person is provided, the device being configured for being worn over a head of the person, comprising:
- ear enclosure means for enclosing the person's ears, such as over-the-ear (OTE) headphones, comprising an audio stimulus device for providing an audio stimulus to one or two of the person's ears;
- virtual reality (VR) glasses for providing a visual stimulus to one or two of the person's eyes; and
- a brain signal measurement device configured for measuring a brain signal of the person in response to the audio stimulus and/or visual stimulus, wherein the determining of the hearing capacity of the person is at least partly based on the measured brain signal.

The above "integrated" device for determining the hearing capacity of the person takes various aspects of the user's response into, i.e. most notably brain signals of the person in response to an audio stimulus and/or visual stimulus. Thus, a much more accurate picture can be obtained of the person's hearing capacity, leading to the prescription of a better-fitting and better-performing hearing aid and a much higher quality of life for the person concerned.

The visual stimulus may be provided to the person by showing pictures or (video) animations or the like.

In case of problems with the middle ear, an audio stimulus may be provided by means of an oscillator e.g. comprised by the VR glasses.

It should be noted that IN 2019/11025478 A discloses a device for detection of hearing loss, in particular a device for early detection of hearing loss in infants using virtual reality (VR) technology, as well as a method of early detection of hearing loss in infants. By delivering quality 3D audio in VR, the device can impart an interactive experience that modifies itself based on both an object's movements and the movements of the user. The device of the present invention works on the principle of 'Doppler effect' and uses a specially designed virtual reality headset for children, i.e. a head-mounted device that provides virtual reality (VR) for the wearer. Head motion tracking sensors are used to capture the rotation of the head, such as gyroscopes, accelerometers and structured light systems. However, IN 2019/11025478 A merely provides a global indication of the wearer's hearing loss in case discrepancies exist between the 3D audio and the VR environment "processed" by the wearer. IN 2019/11025478 A e.g. also does not take brain signals into account.

An embodiment relates to an aforementioned device, wherein the brain signal comprises an electroencephalography (EEG) signal and/or a functional near-infrared spectroscopy (fNIRS) signal. Thus, the auditory pathway can be accurately assessed to determine the person's brain signals in response to the audio and/or visual stimulus. Preferably, a combined fNIRS/EEG system is used to simultaneously measure electrical potentials in brain tissue and changes in brain tissue oxygenation and blood volume.

An embodiment relates to an aforementioned device, wherein the VR glasses comprise an eye signal measurement device for measuring an eye signal of the person in response to the audio stimulus and/or visual stimulus.

The eye signal preferably comprises an electrooculography (EOG) signal, an eye tracking signal and/or an eye movement (EM) signal. Thus, by measuring an aforementioned eye signal of the person, the person's "overall" response to the audio and/or visual stimulus can be better assessed. This is particularly advantageous when investigating possible 3D/environmental audio localization issues. An eye signal or eye tracking may for instance relate to blink duration, blink frequency, eyeball movement, pupil constriction/dilation/size, et cetera.

An embodiment relates to an aforementioned device, wherein the ear enclosure means comprise an ear signal measurement device configured for measuring an ear signal of the person in response to the audio stimulus. Thus, an even more complete picture of the person's hearing capacity can be obtained.

The ear signal measurement device may for instance comprise a light detection and ranging (LiDAR) device for scanning the person's ear canal, such as a volume of the person's ear canal, and/or for measuring movement of the person's eardrum structures, such as the person's eardrum or the tensor tympani muscle (or any other structures connected to the eardrum), in response to the audio stimulus.

An embodiment relates to an aforementioned device, wherein the audio stimulus includes an audiological test, comprising providing audiological test stimuli to the one or two of the person's ears with 8 - 32 frequency channels, at 1/3 octave intervals. Thus, the resolution of the audiological test is greatly increased, compared to the "gold standard" 4 - 8 frequency channels at 1 octave intervals.

An embodiment relates to an aforementioned device, wherein the audio stimulus includes an audiological test, comprising providing audiological test stimuli to the one or two of the person's ears with frequency channels between 20 and 20000 Hz. The resolution of the audiological test is thus even further increased compared to the commonly used 250 - 8000 Hz audiological test.

An embodiment relates to an aforementioned device, wherein the ear enclosure means comprise an audio stimulus device configured for providing an audio stimulus to the two of the person's ears in the form of binaural audio. Thus, the audio stimulus provided to the person is much more realistic, i.e. resembling real-life situations where "spatial" audio stimuli are provided, leading to a much better determination of the hearing capacity of the person and a better-fitting hearing aid.

According to the invention, the ear enclosure means comprise a pair of individual headphones, each one of the pair of individual headphones comprising an audio stimulus device having 6 - 8 speakers and preferably one reference speaker for generating a reference audio signal. Thus, an immersive, realistic, binaural audio stimulus can be provided to the person. The 6 - 8 speakers may each be configured to provide sound in different frequency ranges. The reference audio signal may for instance comprise reference speech, reference music or reference (white) noise. Preferably, the ear enclosure means, such as each one of the pair of individual headphones, are dimensioned, sized or configured to fully comprise or enclose the person's auricle(s). Thus, the acoustics of the auricle can be taken into account when performing measurements. An inner surface of the ear enclosure means may e.g. be spaced at a minimum distance of 3, 4 or 5 mm from the respective auricle.

An embodiment relates to an aforementioned device, wherein the ear enclosure means comprise a pair of individual headphones, each one of the pair of individual headphones having an interior chamber in the form of an icosahedron anechoic chamber. Thus, unwanted sound reflections can be avoided and powerful validation capabilities can be provided over a wide frequency range. Furthermore, a test environment can thus be created, allowing for reproducible and reliable testing.

A method, being not part of the invention, for determining a hearing capacity of a person, using an aforementioned device, is foreseen, comprising the steps of:
- arranging the device over the head of the person;
- providing an audio stimulus to the one or two of the person's ears with the audio stimulus device of the ear enclosure means; and/or
- providing a visual stimulus to the one or two of the person's eyes with the virtual reality (VR) glasses; and
- measuring a brain signal with the brain signal measurement device in response to the audio stimulus and/or visual stimulus;
- determining the hearing capacity of the person, wherein the determining of the hearing capacity is at least partly based on the measured brain signal.

Therein, the VR glasses may comprise an eye signal measurement device for measuring an eye signal of the person in response to the audio stimulus and/or visual stimulus, comprising the further step of:
- measuring the eye signal of the person in response to the audio stimulus and/or visual stimulus.

The ear enclosure means may comprise an ear signal measurement device configured for measuring an ear signal of the person in response to the audio stimulus, comprising the further step of:
- measuring the ear signal of the person in response to the audio stimulus.

In an embodiment, configuring the device for being worn over a head of the person may comprise configuring the device as a helmet.

Preferably, the device is configured to act as a Faraday cage to prevent external electromagnetic fields from interfering with the functioning of the device.

The aforementioned device may furthermore comprise microphones to allow the device to perform active noise cancellation (ANC). Such microphones may be provided in/on an external surface of the device, such as on an external surface of the helmet, when the device is configured as a helmet.

The aforementioned device may also comprise a microphone for recording the person's voice to perform speech analysis. Thus, an even more complete picture of the person's capabilities may be obtained.

Furthermore, the aforementioned ear signal measurement device may be configured to perform Optical Coherence Tomography (OCT) on the one or more ears of the person, such as to determine movement or position of the eardrum.

Also, the aforementioned device may be configured to perform an Electromyography (EMG) on the person, such as the muscles of the person's face or muscles in or around the person's ears, such as to test the stapedius reflex or the functioning of the tensor tympani muscle.

### BRIEF DESCRIPTION OF THE DRAWINGS

The invention will be explained in more detail below, with reference to illustrative embodiments shown in the drawings. Therein:
Figure 1 shows a perspective view of an example embodiment of a device according to the invention arranged on the head of a person;
Figure 2 shows a schematic view of an example embodiment of a device according to the invention, such as the example embodiment shown in Figure 1, arranged on the head of a person;
Figure 3 shows an example of an ear signal measurement device embodied by a LiDAR device in an ear canal of the person;
Figure 4 shows an interior chamber of an individual headphone of a pair of individual headphones, in the form of an icosahedron anechoic chamber;
Figure 5 shows a schematic overview of a method for obtaining and/or analyzing data, using a device according to the invention;
Figure 6 shows a schematic overview of a diagnostic method, using a device according to the invention, as well as diagnostic submethods, device sensors to be used in such submethods, and relevant parameters to be measured with such submethods;
Figure 7 shows a schematic overview of a brain signal measurement device in the form of an fNIRS device;
Figure 8 shows a schematic depiction of the basic functionality of an fNIRS device, such as the fNIRS device of Figure 7;
Figures 9a and 9b show preferred positions of fNIRS emitters and detectors with respect to the brain of the person; and
Figure 10 shows a schematic view of an example embodiment of an ear signal measurement device comprising a LiDAR device according to the invention to be inserted into a person's ear canal.

### DETAILED DESCRIPTION

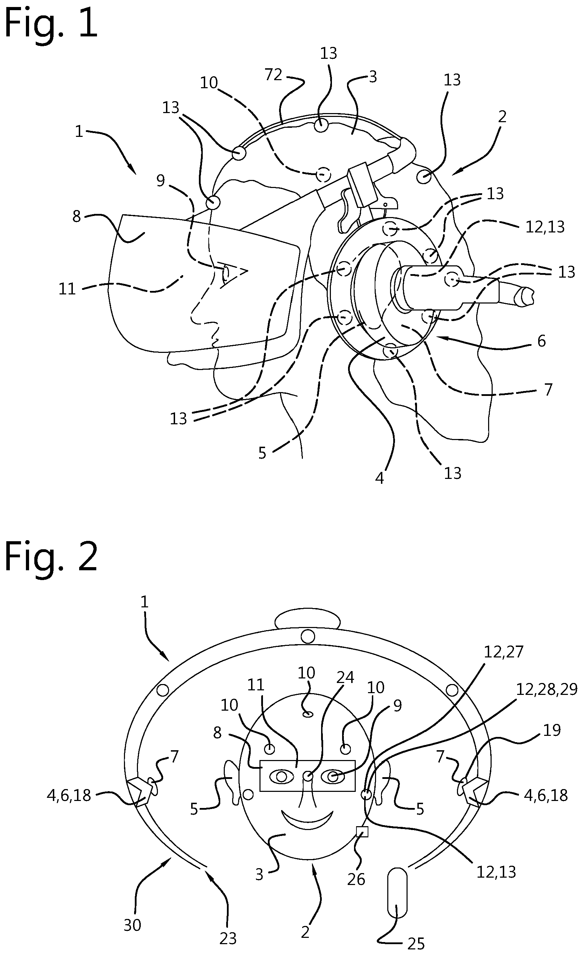

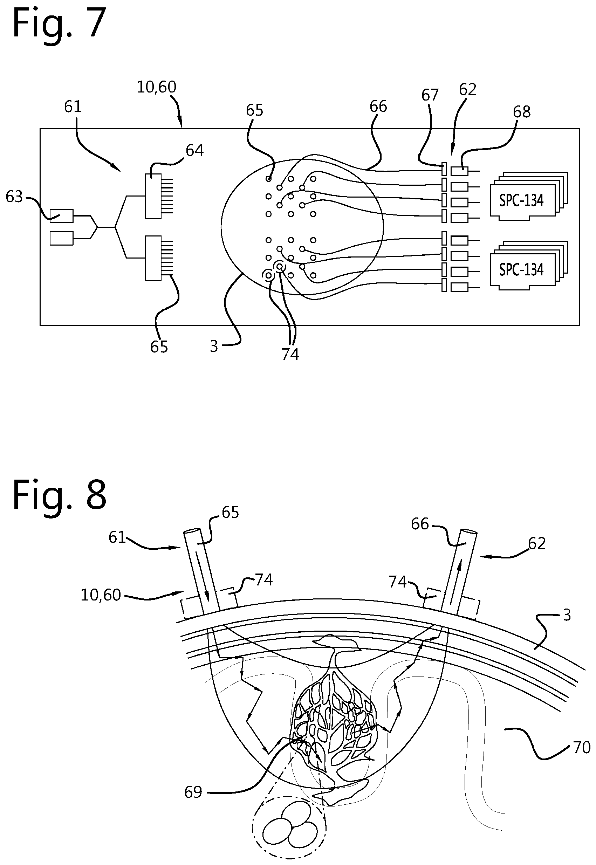

Figure 1 shows a perspective view of an example embodiment of a device 1 for determining a hearing capacity of a person 2 according to the invention arranged on a head 3 of the person 2. The device 1 is configured for being worn over the head 3 of the person and may for instance comprise a helmet (as will be explained with reference to Figure 2). The device 1 comprises ear enclosure means 4 for enclosing the person's ears 5, such as over-the-ear (OTE) headphones 6. The ear enclosure means 4 may have inner dimensions of 80 - 85 mm (height) and/or 40 - 45 mm (width). The ear enclosure means 4 comprise an audio stimulus device 7 for providing an audio stimulus to one or two of the person's ears 5. The audio stimulus device 7 is preferable suitable for producing audio in a frequency range of 100 Hz - 20 kHz. The device 1 furthermore comprises virtual reality (VR) glasses 8 for providing a visual stimulus to one or two of the person's eyes 9. The VR glasses 8 may operate on any conceivable operating system, such as Android or iOS, to name some examples, and may operate using any available engine, as long as the visual output is high-quality (e.g. having 4K resolution). The device 1 also comprises a brain signal measurement device 10 (e.g. attached to the head 3) configured for measuring a brain signal of the person 2 in response to the audio stimulus and/or visual stimulus. Therein, the determining of the hearing capacity of the person 2 is at least partly based on the measured brain signal. The brain signal may comprise an electroencephalography (EEG) signal and/or a functional near-infrared spectroscopy (fNIRS) signal (the latter will be explained in more detail with reference to Figures 7 - 9b). The device 1 may also comprise an ear signal (or ear response) measurement device 12 e.g. in the form a(n) (internal) light detection and ranging (LiDAR) device 13 for scanning the person's ear canal, e.g. in response to the audio stimulus. The device 1 may also comprise several external LiDAR devices 13, e.g. arranged on an external surface of the ear enclosure means 4 and/or on an external surface of the device 1, such as a helmet (e.g. shown in Figure 2), to scan the surroundings of the person 2. The VR glasses 8 may comprise an eye signal (or eye response) measurement device 11 for measuring an eye signal of the person 2 in response to the audio stimulus and/or visual stimulus. The eye signal may comprise an electrooculography (EOG) signal, an eye tracking signal and/or an eye movement (EM) signal. The skilled person will understand that the device 1 may be attached to the head 3 of the person 2 by using appropriate (i.e. comfortable) straps 72, bands, et cetera. The device 1 furthermore should preferably be configured to have sufficient battery life, such as 30 - 36 hours. The device 1 should preferably also be able to communicate via wireless technologies, such as Wi-Fi or Bluetooth. The audio stimulus 7 may for instance comprise ERP, clicking sounds, specific tones, pink noise, music, mono sound, stereo sound, et cetera. The audio stimuli may have an interval of 1.5 seconds - 2.5 seconds, such as 2 seconds. The intensity of the audio stimuli may be 130 dB. Also, in view of the possibly large amounts of data to be communicated/analyzed, the device 1 may comprise photonic chips. The device 1 is preferably also configured to communicate via 5G. The VR glasses 8 may also be used to make people "aware" of hearing loss in a playful manner (e.g. in a VR game setting). Developments with the LiDAR functionality of present-day VR glasses 8 (such as the Oculus Quest) also offer possibilities for hearing diagnostics and optimal adjustment of hearing aids is therefore easier. In particular, linking an environmental LiDAR scan (such as performed with the aforementioned external LiDAR devices 13, e.g. as comprised by the VR glasses 8) with the acoustic value of the environment where the test is carried out, is promising. Such linking can be realized based on algorithmic interpretation. The Applicant foresees that adjusting hearing aids based on the aforementioned acoustic value may help with creating a new generation of smart devices where speech-to-noise ratio is optimized.

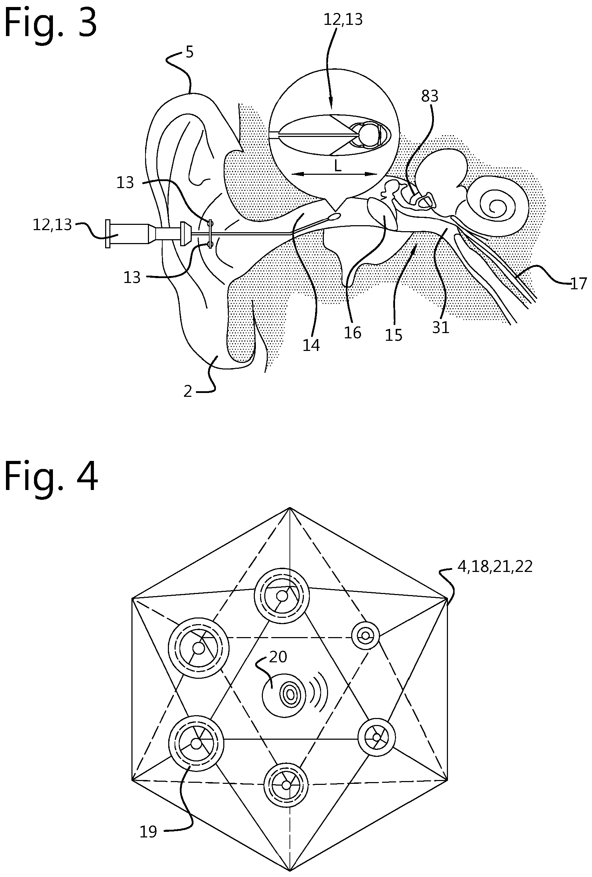

Figure 2 shows a schematic view of an example embodiment of a device 1 according to the invention, such as the example embodiment shown in Figure 1, arranged on the head 3 of a person 2. The device 1 as shown comprises a helmet 23. The helmet 23's outer shell may be made of Kevlar, graphite, carbon fibre, glass fiber, polycarbonate, et cetera. The helmet 23 preferably is configured to protect the person's head 3 against impact in case of an accident. Thereto, an inner shell of the helmet may comprise acoustic fabric panels, acoustic foam panels, acoustic mineral wool, acoustic cotton batts, convoluted acoustic panels, fabric-covered foam panels, or the like, to increase comfort and impact resistance, as well as to increase sound absorption. The device 1 as shown in Figure 2 may have ear enclosure means 4 comprising an ear signal measurement device 12 configured for measuring an ear signal of the person 2 in response to the audio stimulus. As more clearly shown in Figure 3, the ear signal measurement device 12 may comprise a light detection and ranging (LiDAR) device 13 for scanning the person's ear canal 14, such as a volume of the person's ear canal 14, and/or for measuring movement of the person's eardrum structures 15, in response to the audio stimulus, such as the person's eardrum 16, the manubrium malleolaris, the umbo, the musculus stapedius 83 or the tensor tympani muscle 17, to name some examples. The ear signal device measurement device 12 may also comprise an internal microphone 27 (such as an optical microphone). The audio stimulus preferably includes an audiological test, comprising providing audiological test stimuli to the one or two of the person's ears 5 with 8 - 32 frequency channels, at 1/3 octave intervals. The audio stimulus may furthermore include an audiological test, comprising providing audiological test stimuli to the one or two of the person's ears 5 with frequency channels between 100 and 20000 Hz. In case of problems with the middle ear, an audio stimulus may be provided by an oscillator 24 comprised by the VR glasses 8. The device 1 may be configured to perform an Electromyography (EMG) 28 on the person 2, such as the muscles of the person's 2 face or muscles in or around the person's ears 5, such as to test the stapedius reflex or the functioning of the tensor tympani muscle. Alternatively, or in addition thereto, Optical Coherence Tomography (OCT) may be employed. The device 1 may also comprise one or more "skin sensors" 26, e.g. for performing a photoplethysmogram (PPG), i.e. an optically obtained plethysmogram that can be used to detect blood volume changes in a microvascular bed of tissue, such as of the face of the person 2. The one or more skin sensors 26 may also be used to analyze electrodermal activity (EDA), i.e. the property of the human body that causes continuous variation in the electrical characteristics of the skin. The device 1 may comprise an external microphone 25, preferably arranged near the person's 2 mouth, i.e. during use, e.g. for performing speech analysis. The ear signal measurement device 12 may also comprise a nano-camera, i.e. a camera sufficiently small to be inserted into the ear canal of the person 2, or a sufficiently small endoscope. However, a light detection and ranging (LiDAR) device 13 is preferred, because such as device 13 offers a wider range of measurement possibilities. The device 1 may furthermore comprise ANC microphones 30 to allow the device 1 to perform active noise cancellation (ANC). The ANC microphones 30 may be provided in/on an external surface of the device 1, such as on an external surface of the helmet 23. Preferably, the device 1, such as the helmet 23, is configured to act as a Faraday cage to prevent external electromagnetic fields from interfering with the functioning of the device 1. Thereto, the device 1, such as the helmet 23, may comprise aluminum, copper or chicken wire, to name some examples. Preferably, the device 1 also isolates or shields the person 2 (i.e. his or her ears and/or eyes) from light and/or noise.

As mentioned in the foregoing, Figure 3 shows an example of an ear signal measurement device 12 embodied by a LiDAR device 13 in an ear canal 14 of the person 2. As mentioned in the foregoing, the ear signal measurement device 12 may comprise one or more light detection and ranging (LiDAR) device 13 for scanning the person's ear canal 14, such as a volume of the person's ear canal 14, and/or for measuring movement of the person's eardrum structures 15, such as the person's eardrum 16 or the tensor tympani muscle 17 (or any other structures connected to the eardrum), in response to the audio stimulus. For sake of clarity, Figure 3 also shows the middle ear 31.

Figure 4 shows an interior chamber 21 of an individual headphone 18 of a pair of individual headphones 18, in the form of an icosahedron anechoic chamber 22. The ear enclosure means 4, for instance those shown in Figure 1 and 2, may comprise an audio stimulus device 7 configured for providing an audio stimulus to the two of the person's ears 5 in the form of binaural audio. The ear enclosure means 4 comprise a pair of individual headphones 18, each one of the pair of individual headphones 18 comprising an audio stimulus device 7 having 6 - 8 speakers 19 and preferably one reference speaker 20 for generating a reference audio signal. As shown in Figure 4, each one of the pair of individual headphones 18 may have an interior chamber 21 in the form of an icosahedron anechoic chamber 22.

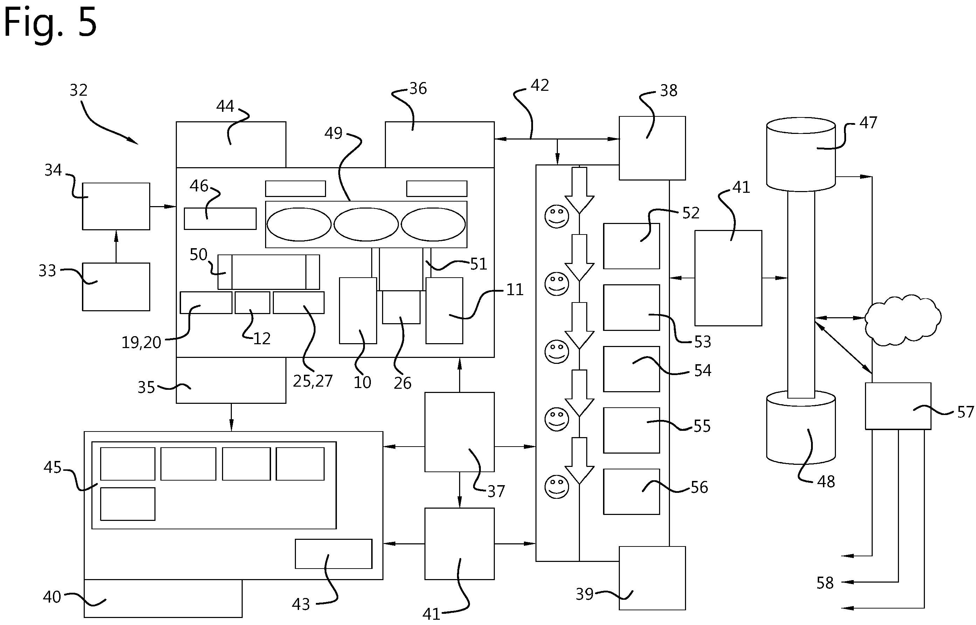

Figure 5 shows a schematic (functional) overview of a method 32 for obtaining and/or analyzing data, such as using a device 1 according to the invention, for establishing the hearing capacity of the person 2. The method 32 (or device 1) may employ an image and sound generator (module) 44 for generating the audio and/or visual stimulus. The image and sound generator 44 may comprise one or more signal generators 29 for generating an audio signal (audio stimulus), an electric signal, and/or a visual signal (visual stimulus). The one or more signal generators 29 may be functionally coupled to a hardware driver 50 and a sensor driver 50 (comprised by the image and sound generator 44). The hardware driver 50 may be used to "drive" one or more speakers 19, 20, an ear signal measurement device 12, e.g. in the form of a camera, and/or a microphone 25 (external) and/or microphone 27 (internal). The one or more signal generators 29 may also be functionally coupled to a sensor driver 51. The sensor driver 51 may be used to "drive" a brain signal measurement device 10, such as employing EEG, ERP or EMG and/or a skin sensor 26, e.g. employing ADR or PPG, and/or an eye signal measurement device 11, e.g. employing EOG, EM, ET or EMG. The one or more signal generators 29 may comprise a microprocessor 46 and may furthermore be functionally coupled to a memory and a transmitter. Preferably, the image and sound generator 44 also utilizes artifact removal and correction, as indicated by reference numeral 36. Prior to activation of the image and sound generator module 44, anamnese/pre-processing may per performed, as indicated by reference numeral 33, as well as prescriptive analytics, as indicated by reference numeral 34, to let the image and sound generator 44 generate the proper audio and/or visual stimuli.

Predictive analytics 35 may then be used to obtain speech data, biological data, visual data, audio data and/or video data. A multiplexer engine 45 may be used for data selection/integration. The multiplexer engine 45 may be part of a cognition and emotion analysis module 40, which may employ a controller 43 for proper functioning.

Real-time feedback may be provided between the artifact removal and correction module 36, or, generally speaking, the image and sound generator module 44 and a data collection/biometric analysis module 38. Data collection/biometric analysis 38 may comprise a scalp data controller 52, a measurement data controller 53, an emotion estimation unit 54, a cognition estimation unit 55, and/or an estimation result output unit 56. A descriptive analytics module 39 is also foreseen. A communication interface 41 between the cognition and emotion analysis module 40 and the data collection/biometric analysis module 38/descriptive analytics module 39 is also provided. A learning data generator and storage 47 and a data storage unit 48 are shown in the right portion of Figure 5. A communication interface 41 is again provided between the data collection/biometric analysis module 38/descriptive analytics module 39 and the learning data generator and storage 47 and a data storage unit 48. The learning data generator and storage 47 is connected to an input/output interface 57, which may provide output 58, for further use in data science, context-aware sensing, et cetera. A feedback artifact removal and correction module 37 may also be provided, functionally connected to the image and sound generator module 44, the cognition and emotion analysis module 40, the (lower) communication interface 41 and the data collection/biometric analysis module 38/descriptive analytics module 39.

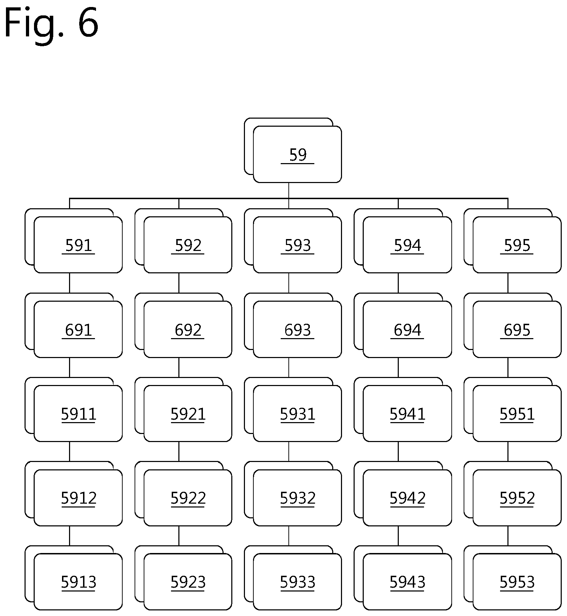

Figure 6 shows a schematic overview of a diagnostic method 59, using a device 1 according to the invention, as well as diagnostic submethods 591 - 595, device sensors 691 - 695 to be used in such submethods 591 - 595, and relevant parameters 5911 - 5953 to be measured with - or specific tests 5911 - 5953 to be performed with - such submethods 591 - 595. Note that neither the diagnostic method 59, nor the diagnostic submethods 591-595 form part of the invention.

Diagnostic submethods 591 - 595 may respectively relate to (from left to right) determining the absolute threshold of hearing (ATH) (591), determining a differential hearing threshold value/frequency and loudness (592), a directional hearing test (593), determining a threshold for speech intelligibility (594), as well as a free field hearing test (595). Device sensors 691 - 695 to be used in such submethods 591 - 595 may respectively comprise (from left to right) ear enclosure means 4/headphones 18 with: internal microphone 27, (nano-)camera, OCT 29 sensors, speaker(s) 19, 20 and/or various other sensors, such as skin sensors 26 (691); VR glasses 8 and ear enclosure means 4/headphones 18 and/or various other sensors (692); VR glasses 8, (nano-)camera, and ear enclosure means 4/headphones 18 and/or various other sensors (693); VR glasses 8, (nano-)camera, and ear enclosure means 4/headphones 18, internal microphone 27, and/or various other sensors (694); VR glasses 8 and REM (695). For diagnostic submethod 591, the specific tests to be performed or parameters to be measured (5911 - 5913) may comprise EEG (5911), PPG 26, EDA 26, heart rate, blood pressure, oxygen saturation, EOG, eye tracking and pupil measurements (5912), and/or EMG 28 (meculus tensor tympani) (5913). For diagnostic submethod 592, the specific tests to be performed or parameters to be measured (5921 - 5923) may comprise EMG 28 and EEG (5921), PPG 26, EDA 26, heart rate, blood pressure, oxygen saturation, EOG, eye tracking and pupil measurements (5922), and EMG 28 (stapedius reflex, tensor tympani), as well as (nano-)camera, OCT 29 (eardrum 16 position) (5923). For diagnostic submethod 593, the specific tests to be performed or parameters to be measured (5931 - 5933) may comprise EEG - peaks Na, Pa, Nb (gyrus temporalis superior, trigeminus), auditive cortex, selective and auditive attention (5931), PPG 26, EDA 26, heart rate, blood pressure, oxygen saturation, EOG, pupil measurements, ear drum 16 movement (5932), and EMG 28 (stapedius reflex, tensor tympani, auricular muscle reflex), (nano-)camera (ear drum 16 position) (5933). For diagnostic submethod 594, the specific tests to be performed or parameters to be measured (5941 - 5943) may comprise EEG (formatio reticularis/thalamus, auditive cortex) - peaks Na, Pa, Nb, superior temporal sulcus, reflexive attention (5941), PPG 26, EDA 26, heart rate, blood pressure, oxygen saturation, EOG, pupil measurements, ear drum 16 movement, speech analysis, attention, S/N ratio, speech synthesis (5942), and EMG 28 (stapedius reflex, tensor tympani, post-auricular muscle reflex), (nano-)camera/OCT 29 (ear drum 16 position) (5943). For diagnostic submethod 595, the specific tests to be performed or parameters to be measured (5951 - 5953) may comprise EEG (formatio reticularis/thalamus, auditive cortex) - peaks Na, Pa, Nb, superior temporal sulcus, reflexive attention, selective and auditive attention (olive superior, inferior colliculus) (5951), PPG 26, EDA 26, heart rate, blood pressure, oxygen saturation, EOG, pupil measurements, ear drum 16 movement, speech analysis, attention, S/N ratio, speech synthesis (5952), and ear canal 14 acoustics, stress and free field S/N ratio (5953).

Figure 7 shows a schematic overview of a brain signal measurement device 10 in the form of an fNIRS device 60. The fNIRS device 60 comprises an fNIRS emitter 61 to emit near-infrared light waves into a blood vessel system of a brain comprised by the person's head 3. The light waves to be emitted by the fNIRS emitters 61 are created by lasers 63. The light waves are transmitted to the head 3 by fibers 65, which are connected to fiber switches 64 for distributing the light waves. An fNIRS detector 62 with ND wheels 67 and detectors 68 then detects the (altered) emitted near-infrared light waves that have travelled through the blood vessel system 69 to estimate cortical hemodynamic activity. One or more of the fibers 65, 66 may be provided with an EEG device 74 arranged on the person's head 3 at the location where the fibers 65, 66 touch the head 3, such that a combined fNIRS/EEG "sensor" is created, for sensing both fNIRS as well as EEG brain signals (also see Figure 8).

Figure 8 shows a schematic depiction of the basic functionality of an fNIRS device 60, such as the fNIRS device 60 of Figure 7, for use as a brain signal measurement device 10. As mentioned before, the fNIRS device 60 comprises an fNIRS emitter 61 to emit near-infrared light waves into a blood vessel system 69 of a brain 70 comprised by the person's head 3 (via a fiber 65). An fNIRS detector 62 then detects the (altered) emitted near-infrared light waves that have travelled through the blood vessel system 69 to estimate cortical hemodynamic activity (via another fiber 66). As shown in Figure 8, the fibers 65, 66 may again be provided with an EEG device 74 arranged on the person's head 3 at the location where the fibers 65, 66 touch the head 3, such that a combined fNIRS/EEG "sensor" is created.

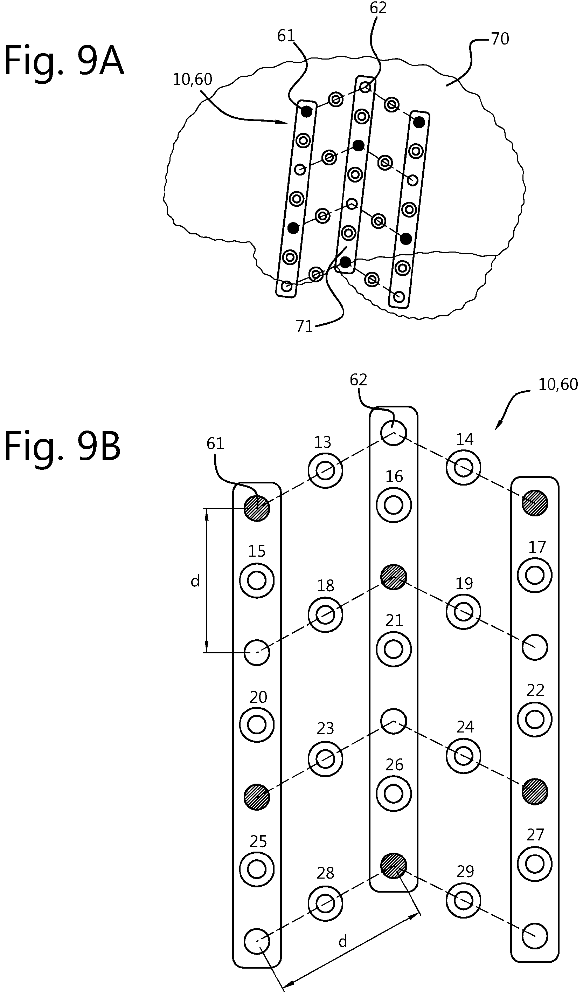

Figures 9a and 9b show preferred positions of fNIRS emitters 61 and detectors 62 with respect to the brain 70 of the person, with the T3 position being indicated by reference numeral 71. The distance "d" may be 25 - 35 mm, such as 30 mm.

A method, not part of the invention, for determining a hearing capacity of a person 2, using an aforementioned device 1, is foreseen. The method comprises the step of arranging the device 1 over the head 3 of the person 2. The method comprises the further step of providing an audio stimulus to the one or two of the person's ears 5 with the audio stimulus device 7 of the ear enclosure means 4 and/or providing a visual stimulus to the one or two of the person's eyes 9 with the virtual reality (VR) glasses 8. The method also comprises the step of measuring a brain signal with the brain signal measurement device 10 in response to the audio stimulus and/or visual stimulus. In addition, the method comprises the step of determining the hearing capacity of the person 2, wherein the determining of the hearing capacity is at least partly based on the measured brain signal.

The VR glasses 8 may comprise an eye signal measurement device 11 for measuring an eye signal of the person 2 in response to the audio stimulus and/or visual stimulus. The method then may comprise the further step of measuring the eye signal of the person 2 in response to the audio stimulus and/or visual stimulus.

The ear enclosure means 4 may comprise an ear signal measurement device 12 configured for measuring an ear signal of the person 2 in response to the audio stimulus. The method then may comprise the further step of measuring the ear signal of the person 2 in response to the audio stimulus.

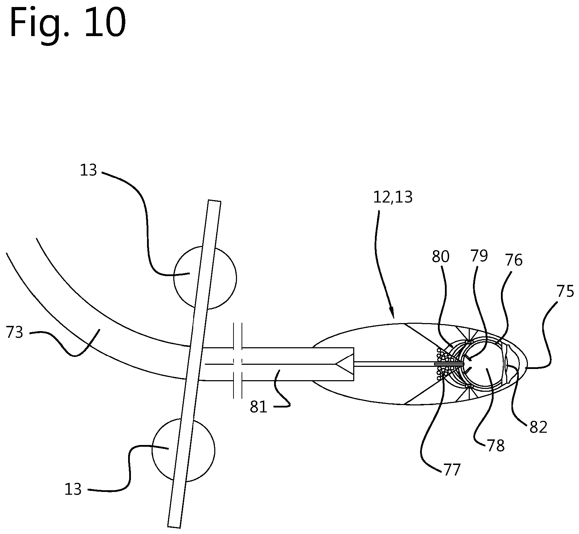

Figure 10 shows a schematic view of an example embodiment of an ear signal measurement device 12 comprising a LiDAR device 13 according to the invention (such as the one shown in Figure 3) to be inserted into a person's ear canal. Two external LiDAR devices 13 are also shown in the left portion of Figure 10. However, these external LiDAR devices 13 are to scan the person's auricle, not the interior of the person's ear canal. The LiDAR device 13 that is to scan the person's ear canal may comprise an aspherical lens 75 at a distal end thereof, as well as an objective lens 82. The LiDAR device 13 may furthermore comprise a mirror telescope 76 comprising water 78, and semi-reflectors 79. The aspherical lens 75 may be provided or may comprise polymethylmethacrylate (PMMA), also known as "Perspex" or "Plexiglas". The LiDAR device 13 may further comprise photodiodes 77 and a polymer 80. A laser 81 connected to an optical fiber 73 provides the necessary laser beam. Thus, the LiDAR device 13 may provide a 270° view of the person's ear canal.

Although the invention has been described above with reference to example embodiments, the invention is defined by the appended claims.

### LIST OF REFERENCE NUMERALS

- 1.: Device for determining a hearing capacity of a person
- 2.: Person
- 3.: Head of the person
- 4.: Ear enclosure means
- 5.: Person's ear
- 6.: Over-the-ear (OTE) headphones
- 7.: Audio stimulus device
- 8.: Virtual reality (VR) glasses
- 9.: Person's eye
- 10.: Brain signal measurement device
- 11.: Eye signal measurement device
- 12.: Ear signal measurement device
- 13.: LiDAR device
- 14.: Person's ear canal
- 15.: Person's eardrum structure
- 16.: Person's eardrum
- 17.: Tensor tympani muscle
- 18.: Individual headphone
- 19.: Speaker
- 20.: Reference speaker
- 21.: Interior chamber
- 22.: Icosahedron anechoic chamber
- 23.: Helmet
- 24.: Oscillator
- 25.: External microphone
- 26.: Skin sensor (PPG/EDA)
- 27.: Internal microphone
- 28.: EMG
- 29.: OCT
- 30.: ANC microphone
- 31.: Middle ear
- 32.: Method for analysis/data processing
- 33.: Anamnese/pre-processing
- 34.: Prescriptive analytics
- 35.: Predictive analytics
- 36.: Artifact removal and correction
- 37.: Feedback artifact removal and correction
- 38.: Data collection/biometric analysis
- 39.: Descriptive analytics
- 40.: Cognition and emotion analysis
- 41.: Communication interface
- 42.: Real-time feedback
- 43.: Controller
- 44.: Image and sound generator
- 45.: Multiplexer engine
- 46.: Microprocessor
- 47.: Learning data generator and storage unit
- 48.: Data storage unit
- 49.: Signal generator
- 50.: Hardware driver
- 51.: Sensor driver
- 52.: Scalp data controller
- 53.: Measurement data controller
- 54.: Emotion estimation unit
- 55.: Cognition estimation unit
- 56.: Estimation result output unit
- 57.: Input/output interface
- 58.: Output (data)
- 59.: Diagnostic method
- 60.: fNIRS device
- 61.: fNIRS emitter
- 62.: fNIRS detector
- 63.: Laser
- 64.: Fiber switch
- 65.: Fiber to head
- 66.: Fiber from head
- 67.: ND wheels
- 68.: Individual fNIRS detector
- 69.: Blood vessel system
- 70.: Brain
- 71.: T3 position
- 72.: Head strap
- 73.: Optical fiber
- 74.: EEG device
- 75.: Aspherical lens
- 76.: Mirror telescope
- 77.: Photodiodes
- 78.: Water
- 79.: Semi-reflectors
- 80.: Polymer
- 81.: Laser
- 82.: Objective lens
- 83.: Stapedius muscle

## Claims

1. Device (1) for determining a hearing capacity of a person (2), the device being configured for being worn over a head (3) of the person, comprising:
- ear enclosure means (4) for enclosing the person's ears (5), comprising an audio stimulus device (7) for providing an audio stimulus to two of the person's ears;
- virtual reality (VR) glasses (8) for providing a visual stimulus to one or two of the person's eyes (9); and
- a brain signal measurement device (10) configured for measuring a brain signal of the person in response to the audio stimulus and/or visual stimulus, wherein the determining of the hearing capacity of the person is at least partly based on the measured brain signal, **characterized in that**
- the ear enclosure means (4) comprise over-the-ear (OTE) headphones (6) with a pair of individual headphones (18), each one of the pair of individual headphones comprising an audio stimulus device (7) having 6 - 8 speakers (19) and preferably one reference speaker (20) for generating a reference audio signal.

2. Device (1) according to claim 1, wherein the brain signal comprises an electroencephalography (EEG) signal and/or a functional near-infrared spectroscopy (fNIRS) signal.

3. Device (1) according to any one of the preceding claims, wherein the VR glasses (8) comprise an eye signal measurement device (11) for measuring an eye signal of the person (2) in response to the audio stimulus and/or visual stimulus.

4. Device (1) according to claim 3, wherein the eye signal comprises an electrooculography (EOG) signal, an eye tracking signal and/or an eye movement (EM) signal.

5. Device (1) according to any one of the preceding claims, wherein the ear enclosure means (4) comprise an ear signal measurement device (12) configured for measuring an ear signal of the person (2) in response to the audio stimulus.

6. Device (1) according to claim 5, wherein the ear signal measurement device (12) comprises a light detection and ranging (LiDAR) device (13) for scanning the person's ear canal (14), such as a volume of the person's ear canal, and/or for measuring movement of the person's eardrum structures (15), such as the person's eardrum (16) or the tensor tympani muscle (17), in response to the audio stimulus.

7. Device (1) according to any one of the preceding claims, wherein the audio stimulus includes an audiological test, comprising providing audiological test stimuli to the one or two of the person's ears (5) with 8 - 32 frequency channels, at 1/3 octave intervals.

8. Device (1) according to any one of the preceding claims, wherein the audio stimulus includes an audiological test, comprising providing audiological test stimuli to the one or two of the person's ears (5) with frequency channels between 20 and 20000 Hz.

9. Device (1) according to any one of the preceding claims, wherein the ear enclosure means (4) comprise an audio stimulus device (7) configured for providing an audio stimulus to the two of the person's ears (5) in the form of binaural audio.

10. Device (1) according to any one of the preceding claims, wherein the ear enclosure means (4) comprise a pair of individual headphones (18), each one of the pair of individual headphones having an interior chamber (21) in the form of an icosahedron anechoic chamber (22).

## Patentansprüche

1. Vorrichtung (1) zum Bestimmen einer Hörfähigkeit einer Person (2), wobei die Vorrichtung dazu ausgelegt ist, über dem Kopf (3) der Person getragen zu werden, umfassend:
- ohrumschließendes Mittel (4) zum Umschließen der Ohren (5) der Person, umfassend eine Akustikreizvorrichtung (7) zum Bereitstellen eines akustischen Reizes an beide Ohren der Person;
- Brille für virtuelle Realität (VR) (8) zum Bereitstellen eines visuellen Reizes an ein oder beide Augen (9) der Person; und
- eine Hirnsignalmessvorrichtung (10), die dazu ausgelegt ist, ein Hirnsignal der Person in Reaktion auf den akustischen Reiz und/oder den visuellen Reiz zu messen, wobei das Bestimmen der Hörfähigkeit der Person wenigstens teilweise auf dem gemessenen Hirnsignal basiert, **dadurch gekennzeichnet, dass**
- das ohrumschließende Mittel (4) einen Über-Ohr (Over-the-Ear, OTE)-Kopfhörer (6) mit einem Paar von Einzelkopfhörern (18) umfasst, wobei jeder von dem Paar von Einzelkopfhörern eine Akustikreizvorrichtung (7) mit 6 - 8 Lautsprechern (19) und vorzugsweise einen Referenzlautsprecher (20) zum Generieren eines Referenzaudiosignals umfasst.

2. Vorrichtung (1) gemäß Anspruch 1, wobei das Hirnsignal ein Elektroenzephalografie (EEG)-Signal und/oder ein funktionelles Nahinfrarotspektroskopie (fNIRS)-Signal umfasst.

3. Vorrichtung (1) gemäß einem der vorstehenden Ansprüche, wobei die VR-Brille (8) eine Augensignal-Messvorrichtung (11) zum Messen eines Augensignals der Person (2) in Reaktion auf den akustischen Reiz und/oder den visuellen Reiz umfasst.

4. Vorrichtung (1) gemäß Anspruch 3, wobei das Augensignal ein Elektrookulografie (EOG)-Signal, ein Augenverfolgungssignal und/oder ein Augenbewegungssignal (EM-Signal) umfasst.

5. Vorrichtung (1) gemäß einem der vorstehenden Ansprüche, wobei das ohrumschließende Mittel (4) eine Ohrsignal-Messvorrichtung (12) umfasst, die dazu ausgelegt ist, ein Ohrsignal der Person (2) in Reaktion auf den akustischen Reiz zu messen.

6. Vorrichtung (1) gemäß Anspruch 5, wobei die Ohrsignal-Messvorrichtung (12) eine Vorrichtung zur Lichtdetektion und Entfernungsmessung (LiDAR) (13) zum Abtasten des Ohrkanals (14) der Person, wie etwa eines Volumens des Ohrkanals der Person, und/oder zum Messen von Bewegungen der Trommelfellstrukturen (15) der Person, wie etwa des Trommelfels (16) der Person oder des Trommelfellspannmuskels (17), in Reaktion auf den akustischen Reiz umfasst.

7. Vorrichtung (1) gemäß einem der vorstehenden Ansprüche, wobei der akustische Reiz einen audiologischen Test beinhaltet, umfassend das Bereitstellen von audiologischen Testreizen an ein oder beide Ohren der Person (5) mit 8 - 32 Frequenzkanälen, in 1/3-Oktav-Intervallen.

8. Vorrichtung (1) gemäß einem der vorstehenden Ansprüche, wobei der akustische Reiz einen audiologischen Test beinhaltet, umfassend das Bereitstellen von audiologischen Testreizen an ein oder beide Ohren der Person (5) mit Frequenzkanälen zwischen 20 und 20000 Hz.

9. Vorrichtung (1) gemäß einem der vorstehenden Ansprüche, wobei das ohrumschließende Mittel (4) eine Akustikreizvorrichtung (7) umfasst, die dazu ausgelegt ist, einen akustischen Reiz an beide Ohren der Person (5) in Form von binauralem Audio bereitzustellen.

10. Vorrichtung (1) gemäß einem der vorstehenden Ansprüche, wobei das ohrumschließende Mittel (4) ein Paar von Einzelkopfhörern (18) umfasst, wobei jeder von dem Paar von Einzelkopfhörern eine Innenkammer (21) in Form einer Ikosaeder-Absorberkammer (22) aufweist.

## Revendications

1. Dispositif (1) pour déterminer la capacité auditive d'une personne (2), le dispositif étant configuré pour être porté sur la tête (3) de la personne, comprenant :
- des moyens d'enveloppement d'oreilles (4) pour envelopper les oreilles (5) de la personne, comprenant un dispositif de stimulus audio (7) pour fournir un stimulus audio à deux des oreilles de la personne ;
- des lunettes (8) de réalité virtuelle (VR) pour fournir un stimulus visuel à un ou deux des yeux (9) de la personne ; et
- un dispositif de mesure de signal cérébral (10) configuré pour mesurer un signal cérébral de la personne en réponse au stimulus audio et/ou au stimulus visuel,
dans lequel la détermination de la capacité auditive de la personne est au moins en partie basée sur le signal cérébral mesuré, **caractérisé en ce que**
- les moyens d'enveloppement d'oreilles (4) comprennent des écouteurs (6) supra-auriculaires (OTE) avec une paire d'écouteurs individuels (18), chacun de la paire d'écouteurs individuels comprenant un dispositif de stimulus audio (7) ayant 6 à 8 haut-parleurs (19) et de préférence un haut-parleur de référence (20) pour générer un signal audio de référence.

2. Dispositif (1) selon la revendication 1, dans lequel le signal cérébral comprend un signal d'électroencéphalographie (EEG) et/ou un signal de spectroscopie fonctionnelle dans l'infrarouge proche (fNIRS).

3. Dispositif (1) selon l'une quelconque des revendications précédentes, dans lequel les lunettes VR (8) comprennent un dispositif de mesure de signal oculaire (11) pour mesurer un signal oculaire de la personne (2) en réponse au stimulus audio et/ou au stimulus visuel.

4. Dispositif (1) selon la revendication 3, dans lequel le signal oculaire comprend un signal d'électro-oculographie (EOG), un signal de suivi oculaire et/ou un signal de mouvement oculaire (EM).

5. Dispositif (1) selon l'une quelconque des revendications précédentes, dans lequel les moyens d'enveloppement d'oreilles (4) comprennent un dispositif de mesure de signal auriculaire (12) configuré pour mesurer un signal auriculaire de la personne (2) en réponse au stimulus audio.

6. Dispositif (1) selon la revendication 5, dans lequel le dispositif de mesure de signal auriculaire (12) comprend un dispositif (13) de détection et de télémétrie par la lumière (LiDAR) pour balayer le conduit auditif (14) de la personne, tel que le volume du conduit auditif de la personne, et/ou pour mesurer le mouvement des structures du tympan (15) de la personne, telles que le tympan (16) ou le muscle tenseur du tympan (17) de la personne, en réponse au stimulus audio.

7. Dispositif (1) selon l'une quelconque des revendications précédentes, dans lequel le stimulus audio comprend un test audiologique, comprenant la fourniture de stimuli de test audiologique à l'une ou aux deux des oreilles (5) de la personne avec 8 à 32 canaux de fréquence, à des intervalles de 1/3 d'octave.

8. Dispositif (1) selon l'une quelconque des revendications précédentes, dans lequel le stimulus audio comprend un test audiologique, comprenant la fourniture de stimuli de test audiologique à l'une ou aux deux des oreilles (5) de la personne avec des canaux de fréquence compris entre 20 et 20000 Hz.

9. Dispositif (1) selon l'une quelconque des revendications précédentes, dans lequel les moyens d'enveloppement d'oreilles (4) comprennent un dispositif de stimulus audio (7) configuré pour fournir un stimulus audio aux deux des oreilles (5) de la personne sous la forme d'un son binaural.

10. Dispositif (1) selon l'une quelconque des revendications précédentes, dans lequel les moyens d'enveloppement d'oreilles (4) comprennent une paire d'écouteurs individuels (18), chacun de la paire d'écouteurs individuels ayant une chambre intérieure (21) sous la forme d'une chambre anéchoïque icosaédrique (22).
